# EUROPEAN PATENT SPECIFICATION

(11) **EP 2 976 155 B1**
(45) Date of publication and mention of the grant of the patent: **21.10.2020**
(21) Application number: 14711276.7
(22) Date of filing: 20.03.2014
(51) Int. Cl.: B01J 37/10, C07C 1/20, C07C 11/04, C07C 11/06, B01J 29/40, B01J 29/48, B01J 29/85

(54) **PROCESS FOR PRODUCING SHORT-CHAIN OLEFINS FROM OXYGENATES**
VERFAHREN ZUR HERSTELLUNG VON KURZKETTIGEN OLEFINEN AUS OXYGENATEN
PROCÉDÉ POUR LA PRODUCTION D'OLÉFINES À CHAÎNE COURTE À PARTIR DE COMPOSÉS OXYGÉNÉS

(30) Priority: 22.03.2013 DE 102013102980
(43) Date of publication of application: 27.01.2016
(73) Proprietor: L'Air Liquide Société Anonyme pour l'Etude et l'Exploitation des Procédés Georges Claude, 75007 Paris (FR)
(72) Inventor: HAAG, Stéphane, 60598 Frankfurt am Main (DE); DROPSCH, Holger, 61130 Nidderau (DE); GORNY, Martin, 65760 Eschborn (DE); OHLHAVER, Theis, 60385 Frankfurt am Main (DE); BACH, Hermann, 56412 Heiligenroth (DE); ROTHÄMEL, Martin, 60437 Frankfurt/Main (DE)
(74) Representative: Dropsch, Holger
(86) International application number: PCT/EP2014/055644
(87) International publication number: WO 2014/147198

(56) References cited:
- EP-A1- 2 460 784
- EP-A1- 2 500 334
- US-A- 4 542 252
- US-A1- 2006 135 834
- US-A1- 2010 210 887

## Description

### Field of the Invention

This invention relates to a process for producing a hydrocarbon product containing short-chain, low-molecular olefins, in particular ethylene and propylene, by conversion of an educt mixture containing steam and oxygenates, for example methanol and/or dimethyl ether (DME), in a multi-stage fixed-bed reactor. The individual stages or reaction zones of the oxygenate-to-olefin synthesis reactor (OTO reactor) are covered with beds of a granular, form-selective zeolite catalyst which before being used in the above-mentioned reaction is subjected to a steam pretreatment. The catalyst can be present as catalytic fixed bed, but also as moving bed in which the catalyst however is not present in the fluidized condition, in contrast for example to fluidized-bed processes.

It is the objective of the process according to the invention to increase the availability of the OTO reactor for the olefin production with the same or an increased yield of short-chain olefins, in particular of propylene (propene), as compared to previously known processes of the same type and thus increase the space-time yield of the reactor with regard to the olefins, in particular with regard to the propylene production.

### Prior art

The production of hydrocarbon mixtures, in particular also of short-chain olefins, by conversion of oxygenates by using form-selective molecular sieve catalysts, in particular of pentasil zeolites of the structure type ZSM-5, is known from the prior art and described for example in the European Patent Application EP 0448000 A1 and the European Patent Specification EP 1289912 B1. The use of multi-stage fixed-bed reactors for this purpose also has been described already. For example, the International Patent Application WO 96/15082 A1 teaches a process for converting a feed mixture containing oxygenate compounds, for example methanol or dimethyl ether, into gasoline-like hydrocarbon compounds in a multi-stage fixed-bed process. In this process, fresh feed material containing oxygenates is supplied to a reaction zone together with the product gas from a preceding reaction zone and additional dilution gas. The temperature and composition of the dilution gas is chosen such that the increase in temperature in the exothermal reaction of the oxygenates to hydrocarbons in each of the succeeding reaction zones is limited to a maximum of 150 °C, wherein the steam partial pressure should not exceed 2.2 ata. In this way, a premature deactivation of the zeolite catalyst used should be prevented, since too high a steam partial pressure at too high temperatures leads to an irreversible change in structure of the zeolite, with which catalytically active centers get lost. On the other hand, steam is required as dilution medium and to prevent excessive carbon deposits on the catalyst. A slow deposition of carbon on the catalyst during the synthesis operation, however, is inevitable. When the same exceeds a tolerable maximum, the production operation must be interrupted and the carbon deposits must be removed for example by controlled burning off. The catalytic activity of the catalyst thereby can largely be restored, i.e. regenerated. The regeneration of the catalyst can be repeated several times, until the above-described irreversible deactivation has decreased the catalytic activity so much that a further use of the catalyst is prohibited for economic reasons. The time interval with production operation of the catalyst between two regenerations is referred to as cycle or also reaction cycle. The first cycle is the operating phase between the restart of the reactor with newly produced catalyst and the first regeneration.

US 4542252 discloses a process for producing a hydrocarbon product containing low-molecular olefins, comprising ethylene and propylene, by conversion of an educt mixture comprising steam and oxygenates, such as methanol and/or dimethyl ether (DME), to olefins in an OTO reactor with several series-connected reaction zones in fluid connection with each other, which are each operated adiabatically and which are filled with a ZSM-5 catalyst, wherein the OTO catalyst is present in the reaction zones as fixed bed or as non-fluidized moving bed,wherein the OTO reactor is charged with the educt mixture and optionally recycle streams and a product stream comprising olefins is discharged from the OTO reactor, characterized in that before being used in the OTO reactor the OTO catalyst is subjected to a steam pretreatment.

The International Patent Application WO 2007/140844 A1 relates to a reactor for producing C₂ to C₈ olefins, preferably propylene, from a feed mixture comprising gaseous oxygenate, preferably dimethyl ether (DME) and/or methanol, steam and one or more of the hydrocarbons, which has a temperature of 400 to 470 °C, and to a method for operating the reactor. The reactor contains a plurality of reaction stages or reaction zones arranged inside a closed upright container, which are traversed by the material stream from top to bottom, each consisting of a supporting tray with a fixed-bed zone located thereon, which is formed of a bed of granular molecular sieve catalyst. In a particular configuration, the reactor contains six reaction zones. Each supporting tray is constructed of cells firmly connected with each other, which are arranged one beside the other without spaces, and is suspended freely in the container. The cells are filled with a layer of molecular sieve catalyst. In the space defined by two adjacent reaction zones at the top and at the bottom, an atomizer system each is provided in the form of a number of nozzle tubes for uniformly spraying a liquid phase containing DME and/or methanol, chiefly consisting of steam and having a temperature of 25 to 150 °C by means of a gas phase saturated with water, chiefly containing DME and/or methanol and having a temperature of 170 to 300 °C towards the reaction stage following next in downstream direction. By spraying the liquid phase, the temperature of the reaction mixture exiting from the reaction stage with a temperature of 400 to 500 °C is lowered to a value of 380 to 470 °C, so that the reaction proceeds in a narrow temperature range (quasi isothermally). The liquid phase can contain up to 30 vol-% of DME and/or methanol and the gas phase can contain up to 80 vol-% of DME and up to 30 vol-% of methanol.

For operating the reactor, a feed mixture containing gaseous oxygenate, preferably DME and/or methanol, as well as steam, which has a temperature of 150 to 300 °C, is cooled to a temperature of 100 to 160 °C, separated into a liquid phase and a gas phase, and liquid phase and gas phase are divided into several partial streams whose number each corresponds to the number of the spaces existing between the reaction stages. Based on a space, a gas-phase partial stream after heating to a temperature of 170 to 300 °C and a liquid-phase partial stream after cooling to a temperature of 25 to 150 °C each is supplied to an atomizer and sprayed into the space. By supplying gas and liquid in a corresponding temperature and quantity between the individual reaction stages, the inlet temperature of the reaction mixture exiting from the reaction stage into the space can be adjusted to the desired temperature before entry into the next following reaction stage.

As stated above, steam is added to the feed mixture as dilution medium during the production of olefins by conversion of oxygenates on form-selective zeolite catalysts. On the other hand, before being used in the oxygenate-to-olefin (OTO) reaction, newly produced zeolite catalysts are subjected to a steam pretreatment, the so-called steaming, as conditioning step. In this steam pretreatment known for example from the European Patent Application EP 0671212 A1 or the US patent specification US 4480145 A, which is carried out after installation of the catalyst in the olefin synthesis reactor and before starting the OTO reaction, the catalyst is charged with steam at a defined temperature and a defined water partial pressure for a precisely determined period. It is the objective of the steam pretreatment to selectively deactivate particularly active, but unselective catalytic centers on the active surface of the catalyst, whereby a long-term stable operation of the catalyst with a good selectivity at the same time for target products such as ethylene and propylene is ensured. When the optimum conditions for the steam pretreatment are exceeded towards the top with regard to temperature, water partial pressure and duration of treatment, irreversible damages of the catalyst can occur, which manifest themselves in losses with regard to activity and selectivity during the succeeding reaction operation. A shortfall of said parameters for the steam pretreatment does not lead to a sufficient conditioning of the catalyst for its operation in the OTO reaction, so that a temporally less stable and less selective reaction operation is observed.

In particular in multistage reactors for carrying out the OTO reaction with several catalyst beds arranged in series as reaction stages, which one after the other are traversed by the feed mixture, carrying out the steam pretreatment turns out to be difficult. A possible cause for this are inhomogeneities with regard to the temperature profile and the distribution of the steam supplied for the steam pretreatment along the length of the OTO reactor, as such reactors often are of the adiabatic type and the steam only can be supplied at the reactor inlet. Therefore, the optimum conditions for the steam pretreatment virtually can be adjusted only for the catalyst bed traversed first, whereas in the subsequently traversed catalyst beds the treatment temperature is lower due to heat losses, so that the conditioning of the catalyst cannot be effected to the required extent. Due to the increasing heat losses, this effect more and more increases in direction towards the reactor outlet. A general increase of the temperature of the steam at the reactor inlet is not possible, as then the catalyst bed traversed first would be subjected to drastic steam pretreatment conditions, which can lead to an irreversible damage of the catalyst.

While the steam pretreatment is carried out, the OTO reactor is not available for carrying out the olefin synthesis. Furthermore, due to the exactly defined treatment conditions during the steam pretreatment the OTO reactor must be heated up step by step after filling in the catalyst and must slowly be approached to the optimum conditions, in order to prevent too strong steaming. The availability of the OTO reactor for the synthesis or production operation thereby is reduced in addition.

### Description of the Invention

The object of the present invention therefore consists in indicating a process for producing a hydrocarbon product containing short-chain, low-molecular olefins, in particular ethylene and propylene, by conversion of an educt mixture containing steam and oxygenates, for example methanol and/or dimethyl ether (DME), in a multistage fixed-bed reactor, which as compared to the processes known from the prior art is characterized by longer cycle times and an increased activity or selectivity of the catalyst to the target products and by an improved temporal availability of the OTO reactor for the reaction and/or synthesis operation.

The aforementioned object is solved with the invention according to claim 1 with a method for producing a hydrocarbon product containing short-chain, low-molecular olefins, comprising ethylene and propylene, by conversion of an educt mixture comprising steam and oxygenates, such as methanol and/or dimethyl ether, under oxygenate conversion conditions to olefins in an oxygenate-to-olefin synthesis reactor (OTO reactor) with several series-connected reaction zones in fluid connection with each other, which are each operated adiabatically and which are filled with a solid, granular, form-selective oxygenate-to-olefin synthesis catalyst (OTO catalyst) on the basis of a zeolite or molecular sieve material, wherein the OTO catalyst is present in the reaction zones as fixed bed or as non-fluidized moving bed, wherein the OTO reactor is charged with the educt mixture and optionally recycle streams and a product stream comprising olefins is discharged from the OTO reactor. The process according to the invention is characterized in that before being used in the OTO reactor the OTO catalyst is subjected to a steam pretreatment and that the steam pretreatment is carried out outside the OTO reactor in a steam pretreatment reactor spatially separate from the OTO reactor under steam pretreatment conditions.

Fluid connection between two reaction zones is understood to be any kind of connection which enables a fluid, for example the educt mixture, to flow from the one to the other of the two regions, regardless of any interposed regions or components.

Short-chain, low-molecular olefins in accordance with the present invention in particular are understood to be olefins which under ambient conditions are present in gaseous form, for example ethylene, propylene as well as the isomeric butenes 1-butene, cis-2-butene, trans-2-butene, iso-butene. Oxygenates are understood to be all oxygen-containing organic compounds which in the process according to the invention can be converted into olefins.

The conversion conditions required for the conversion of oxygenates to olefin products, as well as suitable steam pretreatment conditions, are known to the skilled person from the prior art, for example from the documents discussed above. Necessary adaptations of these conditions to the respective operating requirements will be made on the basis of routine experiments.

Further advantageous aspects of the process according to the invention can be found in sub-claims 2 to 14.

The basic idea underlying the present invention is to carry out the steam pretreatment necessary for conditioning the OTO catalyst not, as is common practice in processes known from the prior art, after installation of the catalyst in the OTO reactor itself, but before charging the OTO reactor with the catalyst in a steam pretreatment reactor spatially separate from the OTO reactor. In the steam pretreatment reactor, the conditioning of the OTO catalyst is effected under steam pretreatment conditions, wherein suitable steam pretreatment conditions are known to the skilled person from the prior art, for example from the documents discussed above. Necessary adaptations of these conditions to the respective operating requirements will be made on the basis of routine experiments. Specific, particularly suitable steam pretreatment conditions will be mentioned below as a particular aspect of the invention.

It is advantageous that by carrying out the steam pretreatment outside the OTO reactor, its availability for the production operation of short-chain olefins correspondingly is higher. In addition, the design of the OTO reactor and that of the steam pretreatment reactor can each be made in an optimum way. Compromises which each lead to a suboptimal reactor design thus are avoided. In particular, the steam pretreatment reactor can be designed smaller than the OTO reactor, so that heating and cooling operations, as they are typical for carrying out the steam pretreatment, are avoided. If the steam pretreatment reactor is equipped with its own heating device, temperature profiles can be imparted to the catalyst filled in for the steam pretreatment, which would not be realizable when carrying out the steam pretreatment in the OTO reactor. The steam pretreatment thus can be carried out under rather isothermal conditions. On the other hand, temporally varying temperature profiles, for example of a ramp-shaped or periodic type, can be imparted to the steam pretreatment reactor, when this is permitted by the heating device used. In this way, the duration of the steam pretreatment possibly can be shorted, or special activity states of the catalyst can be realized, which due to the constructive constraints of the OTO reactor cannot be adjusted when carrying out the steam pretreatment in the OTO reactor.

The removal of the steam-pretreated catalyst from the steam pretreatment reactor after the end of the steam pretreatment and the installation of the catalyst pretreated in this way in the OTO reactor generally is found to be unproblematic, since typical OTO catalysts are not pyrophoric, so that an inertization of the steam-pretreated catalyst before removal from the steam pretreatment reactor generally is not required.

When carrying out the steam pretreatment outside the OTO reactor, a further advantage was found in that in contrast to the conventional steam pretreatment, which is carried out in the OTO reactor, contaminations of the catalyst are avoided. When carrying out the steam pretreatment in the OTO reactor, such contaminations can occur due to the fact that from the synthesis operation preceding the steam pretreatment residues of reactive components, for example methanol or DME, still are present in the reactor feed conduits. During the steam pretreatment in the OTO reactor, the same then get onto the pretreated catalyst, where they lead to undesired carbon deposits and exothermal temperature excursions during the steam pretreatment. This can have a disadvantageous effect on the activity and lifetime of the catalyst.

As a further advantageous effect of the steam pretreatment outside the OTO reactor according to the invention it has been observed that as compared to the synthesis operation with catalysts which were pretreated with steam in the OTO reactor higher selectivities for the target product propylene are achieved more quickly. This is due to the more uniform steam pretreatment of the catalyst pretreated with steam outside the OTO reactor, which leads to a uniformization of the catalytic properties in the various reaction zones arranged in series in the OTO reactor and filled with the steam-pretreated catalyst.

### Preferred Aspects of the Invention

Preferably, the process according to the invention is designed such that the OTO catalyst comprises a material which consists of an alumosilicate zeolite of the pentasil type, preferably of the structure type ZSM-5, or a silicoaluminum phosphate. All of these materials are OTO catalysts known per se. Usually, they are all subjected to the steam pretreatment before carrying out the conversion of the oxygenates to olefins, in order to ensure a more long-term stable catalyst operation.

Preferably, the catalytically active material comprises a zeolite of the structure type ZSM-5. The preferred catalytically active material also can comprise a zeolite of the structure type ZSM-5 which is doped with one or more elements selected from the group including cerium, lanthanum, tungsten, phosphorus, boron. With the preferred catalysts particularly high yields are achieved for short-chain olefins.

When carrying out the process according to the invention, it is advantageous when the treatment temperature during the steam pretreatment lies between 250 and 520 °C, preferably between 450 and 500 °C. At lower temperatures, the desired deactivation of special, highly active catalytic centers does not proceed to a sufficient extent. On the other hand, at higher temperatures too strong a deactivation is effected, so that the catalyst is damaged irreversibly and becomes useless.

Within the indicated temperature range satisfactory degrees of activity of the OTO catalyst are achieved, when
- the weight-related space velocity (WHSV) of the steam stream during the steam pretreatment lies between 0.1 and 100 kg/(kg h), preferably between 0.2 and 10 kg/(kg h), most preferably between 0.4 and 2 kg/(kg h),
- the treatment duration during the steam pretreatment lies between 4 and 250 h, preferably between 10 and 50 h,
- the steam partial pressure during the steam pretreatment lies between 0.1 and 10 bar, absolute, preferably between 0.5 and 5 bar, absolute.

It is possible for the skilled person to determine suitable sets of operating parameters for the steam pretreatment from these indicated ranges by corresponding routine experiments, which parameters are particularly useful for the above-mentioned types of catalyst.

It was found to be particularly advantageous that the steam pretreatment of the entire catalyst quantity required for the reaction operation of the OTO reactor is effected in several individual batches in an isothermally or quasi-isothermally operated steam pretreatment reactor. An isothermal or quasi-isothermal reactor operation is understood to be a mode of operation in which temperature profiles occurring along the longitudinal axis of the steam pretreatment reactor are compensated at least in part, ideally as completely as possible by means of constructive measures. As an example for such constructive measures, the mounting of corresponding support heaters can be mentioned. Both the treatment of the entire catalyst quantity required for the reaction operation of the OTO reactor in several partial batches and the adjustment of temperature conditions as isothermal as possible in the steam pretreatment reactor leads to a particularly strong uniformization of the treatment success over the entire catalyst quantity. The uniformization can additionally be increased in that after completion of the steam pretreatment the individual batches are combined and mixed and partial quantities of these combined and mixed individual batches are charged to the individual reaction zones of the OTO reactor.

A further advantageous aspect of the process according to the invention provides that the steam pretreatment of the OTO catalyst is effected in a steam pretreatment reactor in which the OTO catalyst is present as non-fluidized moving bed. In the case of a horizontal arrangement of the steam pretreatment reactor, for example, the catalyst to be treated can be charged to the same from above by means of a suitable charging device, e.g. a star feeder. The catalyst entering into the steam pretreatment reactor passes through the same from top to bottom driven by gravity, wherein on the bottom side of the steam pretreatment reactor a withdrawal device, for example a second star feeder, is located. The treatment steam is guided in cross flow to the OTO catalyst moving through the steam pretreatment reactor. In this way, the steam pretreatment can be designed as continuous process. Except on treatment temperature, steam space velocity and partial steam pressure, the treatment intensity of the OTO catalyst also depends on the residence time of the catalyst in the steam pretreatment reactor.

In an advantageous development of the invention, a steam pretreatment reactor equipped with catalytic moving beds can be connected with an OTO reactor likewise equipped with catalytic moving beds.

A further advantageous development of the process according to the invention provides that not only the steam pretreatment, but also the catalyst regeneration is carried out outside the OTO reactor. For this purpose, the catalyst deactivated due to carbon deposits must be removed from the OTO reactor and be transferred into the steam pretreatment reactor. The steam pretreatment reactor additionally must be equipped with supply conduits for the regeneration media. When the catalyst regeneration is to be effected by controlled burn-off of the carbon deposits, supply conduits for oxygen or air and for possibly required dilution gases, e.g. nitrogen, must be provided. Furthermore, the waste gases of the regeneration must be supplied to the waste gas disposal via discharge conduits. Subsequent to the regeneration, a steam pretreatment of the regenerated catalyst can be carried out, as far as required, before said catalyst is returned into the OTO reactor. "OTO catalyst" therefore is understood to be both the newly produced catalyst and the catalyst already used before in an OTO reactor. Before being used again in the OTO reactor, the catalyst already used before in an OTO reactor can be subjected to a regeneration.

Particular advantages are obtained when the catalyst both in the OTO reactor and in the steam pretreatment reactor is present as non-fluidized catalytic moving beds, the steam pretreatment reactor additionally is equipped with supply conduits for regeneration media, and the catalyst is circulated between OTO reactor and steam pretreatment reactor. In this way, a completely continuous operation of the process can be ensured with respect to the catalyst circulated between OTO reactor and steam pretreatment reactor, so that interruptions of the synthesis operation, such as for example when carrying out the catalyst regeneration at the end of a cycle, are reduced or even completely avoided. In addition, in the case of the circulation a part of the used catalyst can be discharged from the process continuously and be replaced by newly produced, steam-pretreated catalyst.

### Exemplary embodiments and numerical examples

Further developments, advantages and possible applications of the invention can also be taken from the following description of exemplary embodiments and numerical examples. All features described and/or illustrated form the the invention per se or in any combination, independent of their inclusion in the claims or their back-reference.

The following test results serve to illustrate the effect of the steam pretreatment by the process according to the invention as compared to carrying out the steam pretreatment in the OTO reactor according to the prior art. For this purpose, samples of an OTO catalyst were pretreated with steam in a pilot plant with three identically constructed fixed-bed reactors operated in parallel. To simulate the different extent of the steam pretreatment in dependence on the position of the catalyst bed during the steam pretreatment in the OTO reactor, the three catalyst samples were treated with steam under otherwise identical steam pretreatment conditions for different periods, namely for 48 h, 24 h and 6 h. This corresponds to the first, middle and last catalyst bed in flow direction during the steam pretreatment in the OTO reactor according to the prior art. When starting the steam pretreatment in a technical OTO reactor with several series-connected, adiabatically operated reaction zones, heating is effected by supplying the treatment steam, which has been superheated to the steam pretreatment temperature. The first catalyst bed in flow direction thereby initially is brought to the treatment temperature, whereas the treatment temperature in the downstream catalyst beds only is achieved with a delay, which is the greater the further downstream the catalyst bed is arranged. Consequently, the effective duration of treatment is shortened for the downstream catalyst beds. The catalyst bed arranged in the vicinity of the reactor outlet therefore experiences the shortest effective duration of treatment.

The steam pretreatment for 48 h ("first bed") corresponds to the steam pretreatment method according to the invention in an external steam pretreatment reactor spatially separate from the OTO reactor. As OTO catalyst, the commercially available catalyst MTPROP-1 of Süd-Chemie AG was used, which is based on the zeolite ZSM-5.

Steam pretreatment. The steam pretreatment was effected at 480 °C for the treatment durations indicated above with 1 kg steam / (kg catalyst x h) each, wherein pure steam generated from distilled water was used and the exit of the steam pretreatment reactor was at ambient pressure. The three parallel fixed-bed reactors used as steam pretreatment reactors were designed as identically constructed tubular reactors and were heated by means of a salt bath for adjusting rather isothermal temperature conditions. After completion of the steam pretreatment, the pretreated OTO catalyst samples were cooled to the OTO reaction temperature. Subsequently, the olefin synthesis was started by supplying methanol.

OTO reaction. Under standard operating conditions, a methanol mass flow of 50 g/h each was supplied to the parallel fixed-bed reactors, wherein the catalyst beds were loaded with a space velocity (WHSV) related to the catalyst mass of 1.0 h⁻¹. In addition, the reactor was charged with a constant steam mass flow of 100 g/h. The inlet temperature into the fixed-bed reactors each was 450 °C. The tests were carried out quasi-isothermally, wherein the reactor outlets were at ambient pressure.

In the following Table, the methanol conversions are listed, which were measured during the conversion of methanol to short-chain olefins in dependence on the operating period of the OTO reaction (hos) for the three OTO catalyst samples pretreated with steam under different conditions.

With reference to the data listed in the Table it can be seen that the steam pretreatment of the OTO catalyst by the process according to the invention ("first bed") leads to a greater temporal constancy with regard to the methanol conversions observed during the OTO reaction carried out subsequently. In practice, this means that in operation with OTO catalyst pretreated by the process according to the invention the olefin synthesis reactor can be operated longer as compared to a steam pretreatment of the OTO catalyst *in situ* in the OTO reactor, until an admissible minimum methanol conversion is fallen short of and therefore a regeneration must be carried out. The cycle thus can be prolonged.

**Table: Methanol conversion during the conversion of methanol to short-chain olefins in dependence on the operating period of the OTO reaction (hos).**

| | **48 h steam pretreatment ("first bed") Invention** | **24 h steam pretreatment ("middle bed") Comparative experiment 1** | **6 h steam pretreatment ("last bed") Comparative experiment 2** |
|---|---|---|---|
| **Operating period / hos** | **Methanol conversion / %** | **Methanol conversion / %** | **Methanol conversion / %** |
| 24 | 99.4 | 99.3 | 99.4 |
| 48 | 99.4 | 99.5 | 99.5 |
| 72 | 99.3 | 99.4 | 99.5 |
| 96 | 99.0 | 99.3 | 99.4 |
| 168 | 98.9 | 99.1 | 99.2 |
| 192 | 98.6 | 98.7 | 98.8 |
| 216 | 98.5 | 98.5 | 98.5 |
| 240 | 98.4 | 98.4 | 98.0 |
| 264 | 98.0 | 98.2 | 97.9 |
| 336 | 97.7 | 97.7 | 97.2 |
| 360 | 97.4 | 97.0 | 96.4 |
| 384 | 97.2 | 96.7 | 95.9 |
| 408 | 97.0 | 96.4 | 95.3 |

### Industrial Applicability

With the invention, a process for producing short-chain olefins is proposed, which is characterized by a high temporal constancy of the observed methanol conversions to short-chain olefins, in particular propylene, with a simultaneous prolongation of the cycle time, and thus by a reduced number of regenerations per catalyst charge.

## Claims

1. A process for producing a hydrocarbon product containing short-chain, low-molecular olefins, comprising ethylene and propylene, by conversion of an educt mixture comprising steam and oxygenates, such as methanol and/or dimethyl ether (DME), under oxygenate conversion conditions to olefins in an oxygenate-to-olefin synthesis reactor (OTO reactor) with several series-connected reaction zones in fluid connection with each other, which are each operated adiabatically and which are filled with a solid, granular, form-selective oxygenate-to-olefin synthesis catalyst (OTO catalyst) on the basis of a zeolite or molecular sieve material, wherein the OTO catalyst is present in the reaction zones as fixed bed or as non-fluidized moving bed, wherein the OTO reactor is charged with the educt mixture and optionally recycle streams and a product stream comprising olefins is discharged from the OTO reactor, **characterized in that** before being used in the OTO reactor the OTO catalyst is subjected to a steam pretreatment, and that the steam pretreatment is carried out outside the OTO reactor in a steam pretreatment reactor under steam pretreatment conditions, wherein pure steam generated from distilled water is used for the steam pretreatment.

2. The process according to claim 1, **characterized in that** the OTO catalyst comprises a material which consists of an alumosilicate zeolite of the pentasil type, preferably of the structure type ZSM-5, or a silicoaluminum phosphate.

3. The process according to claim 2, **characterized in that** the material comprises a zeolite of the structure type ZSM-5 which is doped with one or more elements selected from the group including cerium, lanthanum, tungsten, phosphorus, boron,

4. The process according to claim 1, **characterized in that** the treatment temperature during the steam pretreatment lies between 250 and 520 °C, preferably between 450 and 500 °C.

5. The process according to claim 1, **characterized in that** the weight-related space velocity (WHSV) of the steam stream during the steam pretreatment lies between 0.1 and 100 kg/(kg h), preferably between 0.2 and 10 kg/(kg h), most preferably between 0.4 and 2 kg/(kg h).

6. The process according to claim 1, **characterized in that** the treatment duration during the steam pretreatment lies between 4 and 250 h, preferably between 10 and 50 h.

7. The process according to claim 1, **characterized in that** the steam partial pressure during the steam pretreatment lies between 0.1 and 10 bar, absolute, preferably between 0.5 and 5 bar, absolute.

8. The process according to claim 1, **characterized in that** the steam pretreatment of the entire catalyst quantity required for the reaction operation of the OTO reactor is effected in several individual batches in an isothermally or quasi-isothermally operated steam pretreatment reactor.

9. The process according to claim 8, **characterized in that** after completion of the steam pretreatment the individual batches are combined and mixed and partial quantities of these combined and mixed individual batches are charged to the individual reaction zones of the OTO reactor.

10. The process according to claim 1, **characterized in that** the steam pretreatment of the OTO catalyst is effected in a steam pretreatment reactor in which the OTO catalyst is present as non-fluidized moving bed.

11. The process according to claim 10, **characterized in that** as OTO catalyst a newly produced catalyst or a catalyst already used before in an OTO reactor is employed.

12. The process according to any of the preceding claims, **characterized in that** not only the steam pretreatment, but also the catalyst regeneration is carried out outside the OTO reactor.

13. The process according to claims 10 to 12, **characterized in that** both in the OTO reactor and in the steam pretreatment reactor the catalyst is present as non-fluidized catalytic moving beds, the steam pretreatment reactor additionally is equipped with supply conduits for regeneration media, and the catalyst is circulated between OTO reactor and steam pretreatment reactor.

14. The process according to claim 13, **characterized in that** in the case of the circulation a part of the used catalyst continuously is discharged from the process and is replaced by newly produced, steam-pretreated catalyst.

## Patentansprüche

1. Verfahren zur Herstellung eines Kohlenwasserstoffprodukts, enthaltend kurzkettige niedermolekulare Olefine, umfassend Ethylen und Propylen, durch Umwandlung eines Eduktgemisches, umfassend Dampf und Oxygenate wie Methanol und/oder Dimethylether (DME), unter Oxygenatumwandlungsbedingungen zu Olefinen in einem Oxygenat-zu-Olefin-Synthesereaktor (OTO-Reaktor) mit mehreren in Reihe geschalteten Reaktionszonen, die in Fluidverbindung miteinander stehen, die jeweils adiabatisch betrieben werden und die mit einem festen, körnigen, formselektiven Oxygenat-zu-Olefin-Synthesekatalysator (OTO-Katalysator) auf Basis eines Zeolith- oder Molekularsiebmaterials gefüllt sind, wobei der OTO-Katalysator in den Reaktionszonen als Festbett oder als nicht fluidisiertes Fließbett vorhanden ist, wobei der OTO-Reaktor mit dem Eduktgemisch beladen ist und wahlweise Rückführströme und ein Produktstrom, umfassend Olefine, aus dem OTO-Reaktor abgegeben wird, **dadurch gekennzeichnet, dass** der OTO-Katalysator vor seiner Verwendung in dem OTO-Reaktor einer Dampfvorbehandlung unterzogen wird, und dass die Dampfvorbehandlung außerhalb des OTO-Reaktors in einem Dampfvorbehandlungsreaktor unter Dampfvorbehandlungsbedingungen durchgeführt wird, wobei reiner Dampf, der aus destilliertem Wasser erzeugt wird, für die Dampfvorbehandlung verwendet wird.

2. Verfahren nach Anspruch 1, **dadurch gekennzeichnet, dass** der OTO-Katalysator ein Material umfasst, das aus einem Alumosilicatzeolith vom Pentasil-Typ, bevorzugt vom Strukturtyp ZSM-5, oder einem Silicoaluminiumphosphat besteht.

3. Verfahren nach Anspruch 2, **dadurch gekennzeichnet, dass** das Material einen Zeolithen vom Strukturtyp ZSM-5 umfasst, der mit einem oder mehreren Elementen dotiert ist, die ausgewählt sind aus der Gruppe, einschließlich Cer, Lanthan, Wolfram, Phosphor, Bor,

4. Verfahren nach Anspruch 1, **dadurch gekennzeichnet, dass** die Behandlungstemperatur während der Dampfvorbehandlung zwischen 250 und 520° C liegt, bevorzugt zwischen 450 und 500° C.

5. Verfahren nach Anspruch 1, **dadurch gekennzeichnet, dass** die gewichtsbezogene Raumgeschwindigkeit (WHSV) des Dampfstroms während der Dampfvorbehandlung zwischen 0,1 und 100 kg/(kg h) liegt, bevorzugt zwischen 0,2 und 10 kg/(kg) h), am meisten bevorzugt zwischen 0,4 und 2 kg/(kg h).

6. Verfahren nach Anspruch 1, **dadurch gekennzeichnet, dass** die Behandlungsdauer während der Dampfvorbehandlung zwischen 4 und 250 h liegt, bevorzugt zwischen 10 und 50 h.

7. Verfahren nach Anspruch 1, **dadurch gekennzeichnet, dass** der Dampfteildruck während der Dampfvorbehandlung zwischen 0,1 und 10 bar absolut liegt, bevorzugt zwischen 0,5 und 5 bar absolut.

8. Verfahren nach Anspruch 1, **dadurch gekennzeichnet, dass** die Dampfvorbehandlung der gesamten für den Reaktionsbetrieb des OTO-Reaktors erforderlichen Katalysatormenge in mehreren Einzelchargen in einem isotherm oder quasi-isotherm betriebenen Dampfvorbehandlungsreaktor erfolgt.

9. Verfahren nach Anspruch 8, **dadurch gekennzeichnet, dass** nach Beendigung der Dampfvorbehandlung die einzelnen Chargen vereinigt und gemischt werden und Teilmengen dieser vereinigten und gemischten Einzelchargen in die einzelnen Reaktionszonen des OTO-Reaktors geladen werden.

10. Verfahren nach Anspruch 1, **dadurch gekennzeichnet, dass** die Dampfvorbehandlung des OTO-Katalysators in einem Dampfvorbehandlungsreaktor erfolgt, in dem der OTO-Katalysator als nicht fluidisiertes Fließbett vorliegt.

11. Verfahren nach Anspruch 10, **dadurch gekennzeichnet, dass** als OTO-Katalysator ein neu hergestellter Katalysator oder ein bereits zuvor in einem OTO-Reaktor verwendeter Katalysator eingesetzt wird.

12. Verfahren nach einem der vorstehenden Ansprüche, **dadurch gekennzeichnet, dass** nicht nur die Dampfvorbehandlung, sondern auch die Katalysatorregeneration außerhalb des OTO-Reaktors ausgeführt wird.

13. Verfahren nach einem der Ansprüche 10 bis 12, **dadurch gekennzeichnet, dass** sowohl im OTO-Reaktor als auch im Dampfvorbehandlungsreaktor der Katalysator als nicht fluidisierte katalytische Fließbetten vorliegt, der Dampfvorbehandlungsreaktor zusätzlich mit Zufuhrleitungen für Regenerationsmedien ausgestattet ist, und der Katalysator zwischen dem OTO-Reaktor und dem Dampfvorbehandlungsreaktor zirkuliert wird.

14. Verfahren nach Anspruch 13, **dadurch gekennzeichnet, dass** im Falle der Zirkulation ein Teil des verwendeten Katalysators kontinuierlich aus dem Verfahren abgegeben wird und durch einen neu hergestellten, dampfvorbehandelten Katalysator ersetzt wird.

## Revendications

1. Processus pour produire un produit hydrocarboné contenant des oléfines à chaîne courte à faible poids moléculaire, comprenant de l'éthylène et du propylène, par conversion d'un mélange de produits de départ comprenant de la vapeur et des composés oxygénés, tels que le méthanol et/ou le diméthyléther (DME), dans des conditions de conversion de composés oxygénés en oléfines dans un réacteur de synthèse composés oxygénés-oléfines (réacteur OTO) avec plusieurs zones de réaction connectées en série en connexion fluidique les unes avec les autres, qui fonctionnent chacune de façon adiabatique et qui sont remplies avec un catalyseur de synthèse composés oxygénés-oléfines solide granulaire de forme sélectionnée (catalyseur OTO) sur la base d'un matériau de tamis zéolite ou moléculaire, dans lequel le catalyseur OTO est présent dans les zones de réaction en tant que lit fixe ou en tant que lit mobile non fluidisé, dans lequel le réacteur OTO est chargé avec le mélange de produits de départ et facultativement des flux recyclés et un flux de produits comprenant des oléfines est déchargé du réacteur OTO, **caractérisé en ce que,** avant d'être utilisé dans le réacteur OTO, le catalyseur OTO est soumis à un prétraitement à la vapeur, et **en ce que** le prétraitement à la vapeur est exécuté à l'extérieur du réacteur OTO dans un réacteur de prétraitement à la vapeur dans des conditions de prétraitement à la vapeur, dans lequel de la vapeur pure générée à partir d'eau distillée est utilisée pour le prétraitement à la vapeur.

2. Processus selon la revendication 1, **caractérisé en ce que** le catalyseur OTO comprend un matériau qui consiste en un zéolite aluminosilicate du type pentasil, de préférence du type de structure ZSM-5, ou un phosphate de silicoaluminium.

3. Processus selon la revendication 2, **caractérisé en ce que** le matériau comprend un zéolite du type de structure ZSM-5 qui est dopé avec un ou plusieurs éléments sélectionnés à partir du groupe incluant le cérium, le lanthane, le tungstène, le phosphore, le bore,

4. Processus selon la revendication 1, **caractérisé en ce que** la température de traitement durant le prétraitement à la vapeur se trouve entre 250 et 520 °C, de préférence entre 450 et 500 °C.

5. Processus selon la revendication 1, **caractérisé en ce que** la vitesse spatiale liée au poids (WHSV) du flux de vapeur durant le prétraitement à la vapeur se trouve entre 0,1 et 100 kg/(kg h), de préférence entre 0,2 et 10 kg/(kg h), le plus préférentiellement entre 0,4 et 2 kg/(kg h).

6. Processus selon la revendication 1, **caractérisé en ce que** la durée de traitement durant le prétraitement à la vapeur se trouve entre 4 et 250 h, de préférence entre 10 et 50 h.

7. Processus selon la revendication 1, **caractérisé en ce que** la pression partielle de vapeur durant le prétraitement à la vapeur se trouve entre 0,1 et 10 bar, absolue, de préférence entre 0,5 et 5 bar, absolue.

8. Processus selon la revendication 1, **caractérisé en ce que** le prétraitement à la vapeur de la quantité de catalyseur totale nécessaire pour l'opération de réaction du réacteur OTO est effectué dans plusieurs groupes individuels dans un réacteur de prétraitement à la vapeur fonctionnant de manière isotherme ou quasi-isotherme.

9. Processus selon la revendication 8, **caractérisé en ce que,** après l'achèvement du prétraitement à la vapeur, les groupes individuels sont combinés et mélangés et des quantités partielles de ces groupes individuels combinés et mélangés sont chargées sur les zones de réaction individuelles du réacteur OTO.

10. Processus selon la revendication 1, **caractérisé en ce que** le prétraitement à la vapeur du catalyseur OTO est effectué dans un réacteur de prétraitement à la vapeur dans lequel le catalyseur OTO est présent en tant que lit mobile non fluidisé.

11. Processus selon la revendication 10, **caractérisé en ce que,** en tant que catalyseur OTO, un catalyseur nouvellement produit ou un catalyseur déjà utilisé précédemment dans un réacteur OTO est employé.

12. Processus selon l'une quelconque des revendications précédentes, **caractérisé en ce que** non seulement le prétraitement à la vapeur, mais également la régénération de catalyseur sont effectués à l'extérieur du réacteur OTO.

13. Processus selon les revendications 10 à 12, **caractérisé en ce que** à la fois dans le réacteur OTO et dans le réacteur de prétraitement à la vapeur, le catalyseur est présent en tant que lits mobiles catalytiques non fluidisés, le réacteur de prétraitement à la vapeur est de plus équipé de conduits d'alimentation pour des supports de régénération, et le catalyseur circule entre le réacteur OTO et le réacteur de prétraitement à la vapeur.

14. Processus selon la revendication 13, **caractérisé en ce que,** dans le cas de la circulation, une partie du catalyseur utilisé est déchargée en continu du processus et est remplacée par un catalyseur prétraité à la vapeur nouvellement produit.
